# FASCICULE DE BREVET EUROPEEN

(11) **EP 0 530 862 B1**
(45) Date de publication et mention de la délivrance du brevet: **10.04.1996**
(21) Numéro de dépôt: 92119446.0
(22) Date de dépôt: 24.02.1989
(51) Int. Cl.: A61K 7/48, A61K 31/68, A61K 31/07

(54) **Composition cosmétique dermatologique à base de mélanges polyvitaminiques**
Dermatologische kosmetische Zusammensetzung, die Polyvitaminmischungen enthalten
Dermatological cosmetic composition containing polyvitamined mixtures

(30) Priorité: 26.02.1988 LU 87145
(43) Date de publication de la demande: 10.03.1993
(62) Demande divisionnaire de: 89400531.3
(73) Titulaire: L'OREAL, F-75008 Paris (FR)
(72) Inventeur: Griat, Jacqueline, F-94480 Ablon (FR); Soudant, Etienne, F-94260 Fresnes (FR); Zabotto, Arlette, F-75014 Paris (FR); Fanchon, Chantel, F-75015 Paris (FR); Pradier, François, F-75013 Paris (FR)
(74) Mandataire: Stalla-Bourdillon, Bernard

(56) Documents cités:
- GB-A- 2 212 722
- CHEMICAL ABSTRACTS, vol. 73, no. 10, 7 septembre 1970, page 211, résumé no. 48487y, Columbus, Ohio, US; B. ELLOT: "Vitamins (in) cosmetics", & KOSMET.-PARFUM-DROGEN RUNDSCH. 1970, 17(3-4), 33-4, 36-8
- STN FILE SERVER (KARLSRUHE), CHEMICAL ABSTRACTS, vol. 77, no. 24, 1972, page 281, résumé no. 156274t, Columbus, Ohio, US; E. SHERR-BRUZER: "Additives which enhance the efficiency of cosmetics", & OLAJ, SZAPPAN, KOZMET. 1972, 21(1), 18-21

## Description

La présente invention a pour objet une composition cosmétique pour améliorer l'aspect esthétique de la peau, notamment du visage, à l'aide d'associations polyvitaminiques.

Il est connu que certaines associations équilibrées de vitamines permettent de traiter les sujets présentant une déficience de l'état général due à des fatigues physiques ou intellectuelles, au surmenage ou encore à une alimentation déséquilibrée. Par ailleurs, de tels traitements à base de vitamines sont particulièrement recommandés en période de croissance ou de grossesse et en convalescence.

Ces mélanges de vitamines qui peuvent également être associés à des sels minéraux et des oligoéléments contiennent, à des doses thérapeutiquement actives, essentiellement les vitamines suivantes: A, D2, B1, B2, B5, B6, C et E ainsi que de l'acide nicotinique (vitamine PP) et de la biotine (vitamine H). Il est également connu, à la suite de nombreuses études scientifiques, que la peau est un tissu particulièrement sensible aux carences nutritionnelles en vitamines. La carence en vitamines du groupe B, notamment en vitamines B1, B2 et B6 ainsi que la carence en vitamines C et A engendrent des désordres cutanés qui se manifestent essentiellement par un aspect rugueux de la peau, une absence d'élasticité et un manque d'éclat.

En vue de remédier à ces manifestations, il a été proposé des compositions cosmétiques à base de certaines vitamines liposolubles ainsi qu'hydrosolubles dans la mesure où celles-ci étaient suffisamment stables.

L'acide pantothénique (vitamine B5) a également été proposé comme ayant un effet bénéfique sur la peau, bien qu'il n'y ait pas de preuves certaines d'une pénétration à travers l'épiderme.

Ces apports complémentaires de vitamines soit par voie orale, soit par voie topique ne sont généralement pas suffisamment équilibrés en les différentes vitamines de sorte que les traitements à base de compositions polyvitaminiques ne permettent pas d'améliorer l'aspect esthétique de la peau notamment du visage.

A la suite de nombreuses études réalisées dans ce domaine, on a constaté de façon tout à fait surprenante et inattendue que des résultats particulièrement favorables quant à l'aspect esthétique de la peau, pouvaient être obtenus par traitement par voie topique à l'aide d'une composition cosmétique à base d'un mélange approprié de vitamines.

Les résultats obtenus ont en effet permis de mettre en évidence que le traitement selon l'invention améliorait de façon particulièrement significative l'aspect de la peau, celle-ci étant plus lisse, plus douce, et plus souple au toucher.

Par ailleurs, on a constaté que les personnes ainsi traitées avaient dans l'ensemble un visage présentant plus d'éclat et que par ailleurs la peau avait une meilleure résistance aux agressions extérieures.

La présente invention a donc pour objet une composition cosmétique pour améliorer l'aspect esthétique de la peau contenant dans un véhicule approprié, pour une application topique, un mélange de vitamines dans les proportions suivantes par rapport au poids total de la composition :
- Vitamine E : entre 0,3 et 6 %,
- Vitamine A : entre 0,005 et 0,5 %,
- Vitamine B2 : entre 0,001 et 0,01 %,
- Vitamine B5 : entre 0,5 et 3 %, et
- Vitamine H : entre 0,01 et 0,05 %.

La vitamine E ou α-tocophérol, de préférence sous forme d'acétate d'α-tocophérol, est de préférence présente entre 1,5 et 5%.

La vitamine A ou rétinol est de préférence présente entre 0,15 et 0,35%.

La vitamine B2 ou riboflavine, sous forme de son sel de sodium du dérivé 5' phosphate, est de préférence présente entre 0,002 et 0,008%.

La vitamine B5 ou acide pantothénique, de préférence sous forme de D-pantothénate de calcium ou de sodium, est de préférence présente entre 1 et 2%.

La vitamine H ou biotine est de préférence présente entre 0,015 et 0,03%.

Ces différents pourcentages sont ici exprimés par rapport au poids total de la composition cosmétique.

Les études qui ont été réalisées ont permis de mettre en évidence l'importance de ces différentes vitamines en vue de l'effet cosmétique recherché.

La composition cosmétique peut se présenter sous diverses formes permettant une application commode de la composition sur la peau notamment sur le visage.

Ainsi celle-ci peut être par exemple une crème, un lait, un sérum ou encore une émulsion du type huile-dans-l'eau ou eau-dans-l'huile.

Il convient bien entendu que le véhicule de la composition n'ait aucune incidence néfaste sur la stabilité du mélange des vitamines. Les agents émulsionnants de ces compositions sont généralement compris entre 1 et 20 % et de préférence entre 2 et 12 % en poids par rapport au poids total de la composition. Parmi les différents agents émulsionnants qui peuvent être utilisés, on peut en particulier citer :
- les esters d'acides gras polyoxyéthylénés ou polyglycérolés,
- les alcools gras oxyéthylénés ou polyglycérolés comme par exemple l'alcool oléique polyoxyéthyléné à 10 moles d'oxyde d'éthylène, l'alcool stéarylique polyoxyéthyléné de 10 à 20 moles d'oxyde d'éthylène, l'alcool oléique polyglycérolé à 4 moles de glycérol et les alcools gras synthétiques en C₉-C₁₅ polyoxyéthylénés de 5 à 10 moles d'oxyde d'éthylène,
- les sulfates d'alkyle oxyéthylénés ou non, comme le lauryl sulfate de sodium, le lauryl sulfate d'ammonium, le cétyl stéaryl sulfate de sodium, le cétyl stéaryl sulfate de triéthanolamine, le lauryl sulfate de monoéthanolamine, le lauryl éther sulfate de sodium oxyéthyléné (à 2,2 moles d'oxyde d'éthylène par exemple) et le lauryl éther sulfate de monoéthanolamine oxyéthyléné (à 2,2 moles d'oxyde d'éthylène par exemple), et des savons tels que le stéarate de triéthanolamine.

On peut également mentionner le système émulsionnant constitué de lanolate de magnésium et d'alcool de lanoline et/ou de lanoline hydrogénée.

La phase eau des émulsions peut éventuellement contenir des agents gélifiants tels que par exemple l'hydroxypropyl méthylcellulose. La phase huile constituée d'au moins une huile et éventuellement d'au moins une cire, varie entre environ 8 et 50 % en poids par rapport au poids total de la composition.

Parmi les produits gras constituant la phase grasse de ces compositions, on peut citer:
- les huiles hydrocarbonées comme l'huile de paraffine, l'huile de purcellin, le perhydrosqualène et les solutions de cires microcristallines dans les huiles,
- les huiles animales ou végétales comme l'huile d'amande douce, l'huile d'avocat, l'huile de calophyllum, la lanoline, l'huile de ricin, l'huile de cheval, l'huile de porc, l'huile de sésame, l'huile d'olive, l'huile de jojoba, l'huile de karité, l'huile d'hoplostéthus, l'huile de tournesol,
- les huiles minérales dont le point initial de distillation à pression atmosphérique est d'environ 250°C et le point final de l'ordre de 410°C,
- les esters saturés ou non tels que les myristates d'alkyle comme ceux d'isopropyle, de butyle ou de cétyle, le stéarate d'hexadécyle, les palmitates d'éthyle ou d'isopropyle, les triglycérides des acides octanoïque et décanoïque et le ricinoléate de cétyle.

La composition cosmétique peut également contenir d'autres vitamines que celles énumérées, notamment de la vitamine F (acides gras essentiels), de la vitamine D, de la vitamine B6, ou encore l'acide folique ainsi que d'autres substances pour l'entretien et l'amélioration des propriétés de la peau telles que par exemple des humectants, des agents revitalisants et des dérivés protéiniques.

Selon une forme de réalisation particulière, la composition cosmétique contient entre 0,005 et 0,025% d'acide folique et de préférence entre 0,0075 et 0,015%.

Certaines des vitamines de la composition peuvent être incorporées dans des micro-dispersions du type liposomes.

Par ailleurs, la composition peut également contenir des colorants, des parfums, des filtres solaires, des anti-oxydants ainsi que des conservateurs tels que le parahydroxybenzoate de méthyle ou le parahydroxybenzoate de propyle.

Il est préférable d'appliquer la composition cosmétique en effectuant un léger massage afin de la bien faire pénétrer, cette application étant de préférence réalisée le soir de façon à bien laisser agir et pénétrer la composition pendant la nuit.

Les compositions cosmétiques suivantes se sont montrées particulièrement favorables dans la méthode pour améliorer l'aspect esthétique de la peau selon l'invention:

### 1) Crème du type eau-dans-l'huile

| Mélanges de vitamines : | |
|---|---|
| - Vitamine E | 1,5 % |
| - Vitamine A | 0,3 % |
| - Vitamine B2 | 0,003 % |
| - Vitamine B5 | 1 % |
| - Vitamine H | 0,02% |
| - Vitamine F | 2% |
| - Acide folique | 0,008% |
| - Lanolate de magnésium | 7 % |
| - Alcool de lanoline | 3 % |
| - Myristate d'isopropyle | 8 % |
| - Huile de tournesol | 30% |
| - Vaseline | 10 % |
| - Parabydroxybenzoate de méthyle | 0,2 % |
| - Parabydroxybenzoate de propyle | 0,1 % |
| - Eau q.s.p. | 100 % |

### 2) Emulsion du type huile-dans-l'eau

| Mélanges de vitamines : | |
|---|---|
| - Vitamine E | 3 % |
| - Vitamine A | 0,2 % |
| - Vitamine B2 | 0,007 % |
| - Vitamine B5 | 2 % |
| - Vitamine H | 0,03 % |
| - Acide folique | 0,015 % |
| - Perhydrosqualène | 10 % |
| - Polyéthylèneglycol 400 | 3 % |
| - Propylèneglycol | 4 % |
| - Triéthanolamine | 0,6 % |
| - Huile d'amande douce | 2 % |
| - Myristate d'isopropyle | 1 % |
| - Esters d'acides caprique et caprylique et d'alcool gras en C₁₂-C₁₈ | 1 % |
| - Alcool cétylique | 0,5 % |
| - Acide stéarique | 3 % |
| - Mono- et di-stéarate de glycérol non auto-émulsionnables | 3 % |
| - Parfum | 0,1 % |
| - Conservateurs | 0,2 % |
| - Eau q.s.p | 100% |

### 3) Lait du type huile-dans-l'eau

| Mélanges de vitamines : | |
|---|---|
| - Vitamine E | 2 % |
| - Vitamine A | 0,15 % |
| - Vitamine B2 | 0,002 % |
| - Vitamine B5 | 2 % |
| - Vitamine H | 0,015 % |
| - Huile de karité | 3 % |
| - Huile de vaseline | 8 % |
| - Stéarate de glycérol | 2 % |
| - Monostéarate de sorbitan oxyéthyléné à 20 moles d'oxyde d'éthylène (Tween 60) | 1 % |
| - Acide stéarique | 1,4 % |
| - Triéthanolamine | 0,7 % |
| - Carbopol 940 (neutralisé) | 0,2 % |
| - Butylhydroxyanisol/butylhydroxytoluène (50/50) | 0,1 % |
| - Parfum | 1 % |
| - Eau + conservateurs q.s.p. | 100 % |

### 4) Lait du type huile-dans-l'eau

| Mélange de vitamines : | |
|---|---|
| - Vitamine E | 2,50 % |
| - Vitamine A | 0,35 % |
| - Vitamine B2 | 0,008 % |
| - Vitamine B5 | 1,50 % |
| - Vitamine H | 0,02 % |
| - Sesquioléate de sorbitan | 2,00 % |
| - Stéarate de glycérol oxyéthyléné à 20 moles d'oxyde d'éthylène | 2,00 % |
| - Alcool cétylique | 1,00 % |
| - Huile de vaseline | 10,00 % |
| - Huile de jojoba | 2,00 % |
| - Carbopol 940 | 0,20 % |
| - Triéthanolamine | 0,20 % |
| - Parfum | 1,00 % |
| - Parahydroxybenzoate de méthyle | 0,30 % |
| - Eau déminéralisée stérile q.s.p. | 100 % |

## Revendications

1. Composition cosmétique pour améliorer l'aspect esthétique de la peau, caractérisée par le fait qu'elle contient dans un véhicule approprié pour une application topique, un mélange de vitamines dans les proportions en poids suivantes par rapport au poids total de la composition:
- Vitamine E : entre 0,3 et 6%,
- Vitamine A : entre 0,005 et 0,5%,
- Vitamine B2: entre 0,001 et 0,01%,
- Vitamine B5: entre 0,5 et 3%, et
- Vitamine H : entre 0,01 et 0,05%.

2. Composition selon la revendication 1, caractérisée par le fait que le mélange de vitamines est présent dans les proportions en poids suivantes par rapport au poids total de la composition :
- Vitamine E : entre 1,5 et 5%,
- Vitamine A : entre 0,15 et 0,35%,
- Vitamine B2: entre 0,002 et 0,008%,
- Vitamine B5: entre 1 et 2%, et
- Vitamine H : entre 0,015 et 0,03%.

3. Composition selon les revendications 1 et 2, caractérisée par le fait qu'elle contient en outre de la vitamine F, de la vitamine D, de la vitamine B6 et/ou de l'acide folique.

4. Composition selon la revendication 3, caractérisée par le fait qu'elle contient de 0,005 à 0,025% en poids d'acide folique et de préférence de 0,0075 à 0,015%.

5. Composition selon l'une quelconque des revendications précédentes, caractérisée par le fait qu'elle se présente sous forme d'une crème, d'un lait, d'un sérum ou d'une émulsion.

## Claims

1. Cosmetic composition for improving the aesthetic appearance of the skin, characterized in that it contains, in a vehicle suitable for topical application, a mixture of vitamins in the following weight proportions relative to the total weight of composition:
- Vitamin E: between 0.3 and 6%,
- Vitamin A: between 0.005 and 0.5%,
- Vitamin B2: between 0.001 and 0.01%,
- Vitamin B5: between 0.5 and 3%, and
- Vitamin H: between 0.01 and 0.05%.

2. Composition according to Claim 1, characterized in that the mixture of vitamins is present in the following weight proportions relative to the total weight of the composition:
- Vitamin E: between 1.5 and 5%,
- Vitamin A: between 0.15 and 0.35%,
- Vitamin B2: between 0.002 and 0.008%,
- Vitamin B5: between 1 and 2%, and
- Vitamin H: between 0.015 and 0.03%.

3. Composition according to Claims 1 and 2, characterized in that it contains, in addition, vitamin F, vitamin D, vitamin B6 and/or folio acid.

4. Composition according to Claim 3, characterized in that it contains from 0.005 to 0.025% by weight of folic acid, and preferably from 0.0075 to 0.015%.

5. Composition according to any one of the preceding claims, characterized in that it takes the form of a cream, a milk, a serum or an emulsion.

## Patentansprüche

1. Kosmetische Zubereitung zum Verbessern des ästhetischen Aussehens der Haut, dadurch gekennzeichnet, daß sie in einem für die topische Applikation geeigneten Träger ein Gemisch von Vitaminen in den folgenden Gewichtsverhältnissen, bezogen auf das Gesamtgewicht der Zubereitung, enthält:
Vitamin E: zwischen 0,3 und 6 %,
Vitamin A: zwischen 0,005 und 0,5 %,
Vitamin B2: zwischen 0,001 und 0,01 %,
Vitamin B5: zwischen 0,5 und 3 % und
Vitamin H: zwischen 0,01 und 0,05 %.

2. Zubereitung gemäß Anspruch 1, dadurch gekennzeichnet, daß sie das Vitamingemisch in den folgenden Gewichtsverhältnissen, bezogen auf das Gesamtgewicht der Zubereitung, enthält:
Vitamin E: zwischen 1,5 und 5 %,
Vitamin A: zwischen 0,15 und 0,35 %,
Vitamin B2: zwischen 0,002 und 0,008 %,
Vitamin B5: zwischen 1 und 2 % und
Vitamin H: zwischen 0,015 und 0,03 %.

3. Zubereitung gemäß den Ansprüchen 1 und 2, dadurch gekennzeichnet, daß sie zusätzlich Vitamin F, Vitamin D, Vitamin B6 und/oder Folsäure enthält.

4. Zubereitung gemäß Anspruch 3, dadurch gekennzeichnet, daß sie zwischen 0,005 und 0,025 Gew.-% und vorzugsweise von 0,0075 bis 0,015 % Folsäure enthält.

5. Zubereitung gemäß einem der vorstehenden Ansprüche, dadurch gekennzeichnet, daß sie in Form einer Creme, einer Milch, eines Serums oder einer Emulsion vorliegt.
